# EUROPÄISCHE PATENTSCHRIFT

(11) **EP 2 319 822 B1**
(45) Veröffentlichungstag und Bekanntmachung des Hinweises auf die Patenterteilung: **11.01.2017**
(21) Anmeldenummer: 09013922.1
(22) Anmeldetag: 06.11.2009
(51) Int. Cl.: C07C 29/34, C07C 31/125

(54) **Verfahren zur Herstellung von Guerbet-Alkoholen**
Method for producing Guerbet alcohols
Procédé de fabrication d'alcools de Guerbet

(43) Veröffentlichungstag der Anmeldung: 11.05.2011
(73) Patentinhaber: Cognis IP Management GmbH, 40589 Dusseldorf (DE)
(72) Erfinder: Wick, Anja, 40723 Hilden (DE); Mahnke, Eike, Ulf, 42553 Velbert (DE)
(74) Vertreter: BASF IP Association

(56) Entgegenhaltungen:
- WO-A1-91/04242
- US-A- 3 514 493
- P.L. BURK ET.AL.: "The Rhodium promoted Guerbet reaction Part I. Higher alcohols from lower alcohols" J. MOLECULAR CATALYSIS, Bd. 33, 1985, Seiten 1-14, XP002578142
- P.L. BURK ET.AL.: "The Rhodium promoted Guerbet reaction part II. Secondary alcohols and methanol as substrates" J. MOLECULAR CATALYSIS, Bd. 33, 1985, Seiten 15-21, XP002578143

## Beschreibung

### Gebiet der Erfindung

Die Erfindung betrifft ein Verfahren zur Herstellung von Guerbet-Alkoholen.

### Stand der Technik

Fuselöle sind ein Gemisch aus mittleren und höheren Alkoholen (Fuselalkohole), Fettsäureestern, Terpenen und Furfuralen. Sie entstehen bei der alkoholischen Gärung als Nebenprodukte des Hefestoffwechsels und dienen in Bier, Wein und Spirituosen als Geschmacks- und Aromaträger. Beispiele für Fuselalkohole sind Propanole, Butanole, Pentanole (z.B. Isoamylalkohol) und Hexanole. 3-Methyl-1-butanol (ein Isoamylalkohol) ist Hauptbestandteil des Fuselöls. 3-Methyl-1-butanol kann aber nicht nur aus Fuselöl gewonnen werden, sondern z.B. auch durch Hydroformylierung und Reduktion von Buten-Isomeren.

Guerbet-Alkohole sind spezielle verzweigte Alkohole. Es handelt sich um primäre Alkohole, die in beta-Stellung zur CH₂OH-Gruppe verzweigt sind. Guerbet-Alkohole sind dem Fachmann bekannt, manche sind seit langem kommerziell erhältlich. Sie werden durch die so genannte Guerbet-Reaktion gewonnen, eine seit mehr als 100 Jahren bekannte Dimerisierungs-Reaktion, die durch folgendes Formelschema (R* bedeutet darin eine aliphatische Gruppe) umschrieben werden kann:

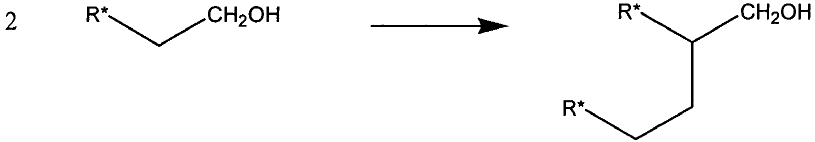

Im Rahmen der klassischen Guerbet-Reaktion wird ein primärer oder sekundärer Alkohol in einen primären Alkohol etwa doppelten Molekulargewichts überführt, der in beta-Position zum C-Atom, das die OH-Gruppe trägt, alkyliert ist. So wird etwa n-Butanol in 2-Ethylhexan-1-ol überführt, Hexan-1-ol in 2-Butyloctan-1-ol und Octan-1-ol in 2-Hexyl-dodecan-1-ol.

Die für die Guerbet-Reaktion eingesetzten primären oder sekundären Alkohole tragen an dem C-Atom, das dem C-Atom mit der OH-Gruppe unmittelbar benachbart ist, mindestens ein Wasserstoffatom - in vielen Fällen tragen sie zwei Wasserstoffatome, d.h. dem C-Atom mit der OH-Gruppe ist dann eine Methylengruppe unmittelbar benachbart.

Das entstandene Kondensationsprodukt kann mit noch in der Reaktionsmischung vorhandenem Ausgangsalkohol weiterreagieren, wobei eine Reihe weiterer Alkohole mit höherem Molekulargewicht entsteht. In welchem Ausmaß diese Nebenreaktion abläuft hängt im Einzelfall von der Art der Ausgangsalkohole und den Reaktionsbedingungen ab. Ferner können weitere Nebenreaktionen ablaufen, die zu Aldehyden, Ketonen, Carbonsäure oder Carbonsäureestern als Nebenprodukten führen. US 3,979,466 führt hierzu (vergl. dort Spalte 1, Zeilen 32-35) aus: "It is further indicated that a plurality of different reactions are likely involved so that the process is highly sensitive and unpredictable as to the effect of particular steps."

Die Guerbet-Reaktion verläuft typischerweise in Gegenwart einer Base bei erhöhter Temperatur unter Wasserabspaltung und stellt eine einfache Möglichkeit dar, lineare Alkohole in verzweigte Alkohole zu überführen. Typischerweise setzt man nur einen einzigen Alkohol bei der Guerbet-Reaktion ein. Es ist jedoch auch möglich, zwei unterschiedliche Alkohole einzusetzen; in diesem Falle spricht man von einer gemischten Guerbet-Reaktion. Die historisch erste Reaktion der genannten Art wurde von Marcel Guerbet bereits im Jahre 1899 publiziert, der n-Butanol zu 2-Ethylhexan-1-ol dimerisiert hatte.

Anthony J. O'Lenick beschreibt in Journal of Surfactants and Detergents, Vol. 4 (2001), S. 311-315, dass im Zuge der Gesamtreaktion mehrere Teilschritte ablaufen und zwar (a) Oxidation des Ausgangsalkohols zum Aldehyd, (b) Aldol-Kondensation, (c) Dehydratisierung (Wasserabspaltung) zu einem ungesättigten Aldehyd und (d) Hydrierung des allylischen Aldehyds.
Nach O'Lenick sind folgende Informationen über die Teilschritte bekannt: (1) Die Reaktion kann im Prinzip ohne Katalysator ablaufen, wird jedoch stark durch die Gegenwart eines Wasserstoff-Transfer-Katalysators beschleunigt. (2) Bei "tieferen" Temperaturen (130 bis 140°C ist der Oxidationsprozess, also die intermediäre Aldehyd-Bildung der geschwindigkeitsbestimmende Schritt. (3) Bei etwa höheren Temperaturen (160 bis 180 °C) ist die Aldol-Kondensation der geschwindigkeits-bestimmende Schritt. (4) Bei noch höheren Temperaturen werden Nebenreaktionen dominant.

Bereits seit den 60-er und 70-er Jahren des 20. Jahrhunderts wird die Guerbet-Reaktionen zur Herstellung kommerzieller Produkte typischerweise so durchgeführt, dass man basische Katalysatoren einsetzte, in der Regel Natrium- oder Kaliumhydroxid. Häufig wird bei der Guerbet-Reaktion neben der Base ein zusätzlicher Katalysator eingesetzt, in der Praxis meist Zinkoxid.

Gemäß US 3,119,880 können Alkalimetallhydroxide, Bleiacetat und Nickel auf Kieselgur eingesetzt werden, wobei Nickel als Dehydrierungs-Katalysator dient.

Gemäß US 3,979,466 werden Alkalikatalysatoren in Kombination Mit Palladium-(II)-Katalysatoren eingesetzt.

WO 91/04242 beschreibt einen verbesserten Guerbet-Prozess, bei dem Alkohole in Gegenwart einer Base und einer Carbonylverbindung bei Temperaturen oberhalb von 180 °C dimerisert werden. Als Startalkohole werden dabei Alkohole mit 4 bis 22 C-Atomen eingesetzt, wobei Alkohole mit 6 bis 18 C-Atomen bevorzugt sind. Die Beschreibung erwähnt auf S.9 auch die Möglichkeit, zusätzlich einen Co-Katalysator, z.B. Komplexe oder Salze von Al, Ni, B, Mg, Cu, Zn, Ti, Zr oder einem Edelmetall der Gruppe VIII, insbesondere Pt, Pd, Rh, Ir und Ru, zu verwenden, ohne dies in den Beispielen zu exemplifizieren.

Carlini et al. beschreiben in Journal of Molecular Catalysis A (2004), S. 65-70 die Herstellung von 2-Ethyl-hexanol aus Butanol durch Guerbet-Reaktion bifunktioneller Katalysatoren auf Cu- oder Pd-Basis und Natriumbutanolat. Sie erwähnen dabei zunächst für den ganz speziellen Fall einer Umsetzung von Methanol mit n-Propanol zu i-Butanol den Einsatz eines Pd/C-Katalysators in Kombination mit Natriumbutanolat bei Temperaturen über 200 °C (Seite 66, linke Spalte, unten). Auf den Seiten 67-69 berichten Sie dann über ihre Untersuchungen zur Selbstkondensation von Butanol bei 200 °C unter Pd(II)- und Pd(0)-Katalyse in Kombination mit Natriumbutanolat-Katalyse. Die Reaktionen wurden in einem 300 ml Reaktor durchgeführt, wobei eine Menge von etwa 0,5 mol Butanol zum Einsatz kam. Tabelle 1 auf S. 68 fasst die Versuchsdaten zusammen. Carlini untersuchte auch, inwieweit der eingesetzte Katalysator unter den Reaktionsbedingungen "stabil" blieb. Dabei fand er heraus, dass es zu "solid deposition and leaching effects" kam, dass also der heterogene Katalysator sich zum Teil an den Reaktorwandungen niederschlug und zum Teil in Lösung ging. Das Leaching (Auslaugen des eingesetzten heterogenen Katalysators) erwies sich als beträchtlich. Carlini stellte fest, dass 50% des eingesetzten Palladiumkatalysators in Lösung gingen (vergl. S. 69, linke Spalte, erster Absatz). Carlini kommt zu folgendem Schluss: "This high leaching extent clearly reduces the interest for industrial application perspectives of heterogeneous palladium-based systems". Carlinis Schlussfolgerung bedeutet, dass er dem Fachmann zumindest für industrielle Anwendungen davon abrät, Palladium-basierte Katalysatorsysteme bei Guerbetreaktionen einzusetzen, weil es hier bedingt durch das Leaching zu einem außerordentlich hohen Verlust der Aktivsubstanz des Katalysators kommt. Matsu-ura et al. beschreiben in Journal of Organic Chemistry (2006), S. 8306-8308 die Guerbet-Reaktion unter Iridium-Katalyse in Gegenwart von Alkenen und Basen. In dem die beiden Spalten von S. 8307 überbrückenden Absatz wird beschrieben, dass sich unter diesen Bedingungen 3-Methyl-1-butanol (ein Isoamylalkohol) in 50%-iger Ausbeute dimerisieren läßt (vergleiche Eintrag 9 der Tabelle 2, S. 8307 rechte Spalte). Das Dimerisierungsprodukt hat die Struktur:

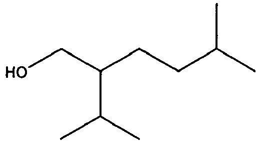

Aufwändig und somit nachteilig an dem Verfahren nach Matsu-ura ist die Notwendigkeit, in Gegenwart eines Alkens, das als Wasserstoff-Akzeptor dient, zu arbeiten. Als Iridium-haltige Katalysatoren wurden [IrCl(cod)]₂ oder [Cp*IrCl₂]₂ eingesetzt.
WO 2009/081727 A1 beschreibt die Dimerisierung von Alkoholen mit maximal 4 C-Atomen. Dabei wird die Guerbet-Reaktion in Gegenwart von Komplexen von Übergangsmetallen und einer Base durchgeführt. Dabei beträgt der Wasserstoffpartialdruck mindestens 0,1 MPa.

Burk et.al. beschreiben im Journal of Molecular Catalysis, 33 (1985) 15-21 die Rhodium katalysierte Guerbet Reaktion von sekundären Alkoholen mit Methanol.

### Beschreibung der Erfindung

Aufgabe der Erfindung war es, ein verbessertes Verfahren zur Dimerisierung von Alkoholen im Sinne einer Guerbet-Reaktion bereitzustellen. Dabei sollte die Guerbetreaktion mit guten Ausbeuten verlaufen und mit geringer Nebenproduktbildung. Die Verfahrensbedingungen sollten im Vergleich zum Stand der Technik mindestens gleichwertig, eher jedoch günstiger sein; dies bedeutet insbesondere, dass die Guerbet-Reaktion unter vergleichweise günstigen Temperatur- und Druckbedingungen verlaufen soll. Ferner sollten eingesetzte Katalysatoren möglichst Ressourcen-schonend einsetzbar sein, d.h. Katalysatorverluste sollten sich in technisch vertretbaren Grenzen halten.

Die vorliegende Erfindung betrifft ein Verfahren zur Dimerisierung von Alkoholen im Sinne einer Guerbet-Reaktion, bei dem man 3-Methyl-butan-1-ol, in Gegenwart
(a) einer Base (B),
(b) einer Carbonylverbindung (C) und
(c) Palladium als Hydrierkatalysator (H) wobei dieses Metall in elementarer Form vorliegen muss, umsetzt.

Bei der Guerbet-Reaktion kann ein einziger Ausgangsalkohol eingesetzt werden; es können aber auch mehrere unterschiedliche Alkohole als Ausgangsalkohole eingesetzt werden. Als Hauptprodukt der Guerbet-Reaktion entstehen Dimerisierungsprodukte, jedoch in untergeordneten Mengen auch höhere Homologe, insbesondere Trimerisierungsprodukte, die daraus resultieren, dass die primären Dimerisierungsprodukte mit noch nicht umgesetzten Ausgangsalkoholen (MA) ebenfalls im Sinne einer Guerbet-Reaktion reagieren.

Wenn daher von einem "Verfahren zur Dimerisierung von Alkoholen im Sinne einer Guerbet-Reaktion" die Rede ist, schließt dies neben der Bildung von Dimerisierungsprodukten auch die die Bildung höherer Homologer ein.

Setzt man ausschließlich einen primären oder sekundären Monoalkohol der Formel (MA) ein, so handelt es sich um die "klassische" Form der Guerbet-Reaktion; setzt man mehrere primäre und/oder sekundäre Monoalkohole der Formel (MA) ein, so handelt es sich um eine "gemischte" Guerbet-Reaktion.

### Zu den Ausgangsalkoholen (A)

Wie ausgeführt setzt man die oben genannte Verbindung (A) 3-Methyl-butan-1-ol als Ausgangsalkohol für das erfindungsgemäße Verfahren ein.

### Zu den Basen (B)

Die Auswahl der beim erfindungsgemäßen Verfahren einzusetzenden Basen (B) ist an sich nicht kritisch.

Beispiele geeigneter Basen (B) sind Alkali- und Erdalkalihydroxide, Alkali- und Erdalkalicarbonate, Alkoxide, Amide und Hydride. Vorzugsweise setzt man Alkalihydroxide ein, insbesondere Kaliumhydroxid.

In einer besonders bevorzugten Ausführungsform setzt man solche Basen (B) ein, bei denen Kalium als Kation fungiert. Beispiele sind Kaliumalkoholate und Kaliumhydroxid. Kaliumhydroxid ist dabei ganz besonders bevorzugt, insbesondere in Form einer wässrigen Lösung, z.B. als 50%-ige wässrige KOH.

Vorzugsweise setzt man die Verbindungen (B) in einer Menge von 0,5 bis 12 Gew.-%, vorzugsweise 1,5 bis 7 Gew.-% - bezogen auf die eingesetzten Ausgangsalkohole - ein.

Die Art der Zugabe der Base (B) ist an sich nicht kritisch und kann auf verschiedene Weise erfolgen, die der Fachmann routinemäßig auf den Einzelfall hin optimieren kann. In einer Ausführungsform wird die Gesamtmenge der Base (B) gleich zu Beginn der Guerbet-Reaktion zugesetzt. Insbesondere bei kurzkettigen Alkoholen kann es günstiger sein, die Base (B), insbesonder KOH, portionsweise zuzudosieren. Vorzugsweise wird bei der portionsweisen Zudosierung der Base (B) - insbesondere KOH - die Guerbet-Reaktion mit einer dem Fachmann bekannten Druckrampe gefahren, um das Reaktionsgemisch bei konstanter Temperatur nahe des Siedepunktes zu halten. Die beschriebenen Varianten der portionsweisen Zudosierung der Base (B) und der Einsatz einer Druckrampe führt meist zu einer signifikanten Ausbeutesteigerung.

### Zu den Carbonylverbindungen (C)

Die Auswahl der beim erfindungsgemäßen Verfahren einzusetzenden Carbonylverbindungen (C) ist an sich nicht kritisch.

Sofern man primäre Ausgangsalkohole (MA) einsetzt, setzt man als Carbonylverbindungen (C) vorzugsweise Aldehyde ein, insbesondere solche, die von den eingesetzten Ausgangsalkoholen (MA) dadurch ableiten, dass man formal die Alkoholgruppe durch eine Aldehydgruppe ersetzt.

Vorzugsweise setzt man die Verbindungen (C) in einer Menge von 0,01 bis 10 Gew.-%, vorzugsweise 0,5 bis 5 Gew.-% - bezogen auf die eingesetzten Ausgangsalkohole - ein.

### Zu den Hydrierkatalysatoren (H)

Der einzusetzende Hydrierkatalysator (H) ist Palladium, wobei dieses Metall in elementarer Form vorliegen muss.

Unter dem Ausdruck "in elementarer Form" ist zu verstehen, dass die Metalle in Form der Elemente der Oxidationsstufe null vorliegen, und zwar in Form der reinen Metalle, jedoch nicht in Form von Verbindungen oder Komplexen der Metalle. Dies impliziert klar, dass Salze oder Komplexe oder Verbindungen dieser Metalle nicht unter die Definition der Hydrierkatalysatoren (H) gemäß der vorliegenden Erfindung fallen. Logischerweise fallen Metall-Ligand-Komplexe oder Metall-Verbindungen auch dann nicht unter die Definition der Hydrierkatalysatoren (H) gemäß der vorliegenden Erfindung, wenn in ihnen das Metall die Oxidationssrufe null aufweist.

Das genannte Metall ist ein Edelmetall der Gruppe VIII des Periodensystems. Es muss in elementarer Form vorliegen und ist somit ein heterogener Katalysator.

Mithin fallen Salze dieses Metalls oder Komplexe dieses Metalls nicht unter die Definition der Komponente (H) gemäß der vorliegenden Erfindung, auch nicht Komplexe dieses Metalls, bei denen das Metall in der Oxidationsstufe 0 vorliegt.

In einer bevorzugten Ausführungsform ist das Palladium, eine hydrophobe Umgebung eingebunden. Das kann etwa dadurch realisiert sein, dass das Metall auf Kohle oder auf modifizierter Kieselsäure oder hydrophobisierter Kieselsäure immobilisert vorliegt. Die Immobilisierung und Hydrophobisierung kann auch auf anderen modifizierten mineralischen Gerüstsubstanzen wie

Zeolithen Hydrotalciten oder Silicaten erfolgen. Dass das Palladium in eine hydrophobe Umgebung eingebunden ist, insbesondere auf hydrophobe Träger aufgebracht ist, hat den Vorteil, dass es vor dem Auswaschen durch Wasser und der Deaktivierung durch alkalische Verbindungen weitgehend geschützt ist.

Insbesondere dann, wenn die Reaktionstemperatur unterhalb von 200 °C und insbesondere unterhalb von 190 °C liegt, ist ein möglicher Auswasch-Effekt ("leaching"), der ja einen Verlust teuren Katalysators bedeutet und daher verfahrenstechnisch unökonomisch ist, auf diese Weise für einen technischen Prozess vertretbar gering.

Palladium auf Kohle (Pd/C) ist als Hydrierkatalysatoren (H) ganz besonders bevorzugt. Vorzugsweise setzt man den Katalysator (C) in einer Menge von 0,0005 bis 0,1 Gew.-%, vorzugsweise 0,001 bis 0,008 Gew.-% - Metallgehalt des Katalysators (C) bezogen auf die eingesetzten Ausgangsalkohole - ein.

### Verfahrenstechnische Parameter

Die optimale Reaktionstemperatur zur Herstellung von Guerbetalkoholen ist gemäß dem Stand der Technik relativ hoch anzusetzen; typischerweise wird im Bereich von 230 bis 280°C und insbesondere 245-280°C gearbeitet. Bei der Herstellung von insbesondere kurzkettiger Guerbetalkoholen mit weniger als 16 C-Atomen werden in diesem Temperaturbereich Dampfdrücke von mehr als 10 bar erreicht, die technisch sehr hohe Anforderungen an die Anlagen stellen und daher schwer zu realisieren sind. Das erfindungsgemäß einzusetzende Katalysatorsystem hat demgegenüber den Vorteil, dass es gestattet, unter milderen Reaktionsbedingungen zu arbeiten: So kann beim erfindungsgemäßen Verfahren in Abhängigkeit des eingesetzten Ausgangsalkohols bei 120-250°C gearbeitet werden. Vorzugsweise arbeitet man bei 140 bis 230 °C. Durch die genannte Absenkung der Reaktionstemperatur werden auch niedrigere Drücke erhalten, die eine technische Realisierung auch kurzkettiger Guerbetalkohole in üblichen technischen Anlagen (pₘₐₓ = 6 bar) ermöglichen. Vorzugswiese arbeitet man bei Drücken von 0,01 bis 15 bar und insbesondere 0,1 bis 6 bar.

Insgesamt zeichnet sich das erfindungsgemäße Verfahren durch mehrere Vorteile aus:
- Die Guerbetreaktion verläuft mit guten Ausbeuten.
- Die Nebenproduktbildung ist gering.
- Die Reaktionstemperaturen und Drücke sind im Vergleich zum Stand der Technik niedriger.
- Der Hydrierkatalysator (H) ist dann besonders gut gegen Auswaschen durch Wasser und vor alkalischen Verbindungen geschützt, wenn das elementare Metall (Palladium) in eine hydrophobe Umgebung eingebunden ist.
- Die Reaktion verläuft unter vergleichsweise günstigen Temperatur- und Druckbedingungen.

### Beispiele

### Eingesetzte Materialien:

Pd/C: 5% Pd/C 3610 von der Firma Johnson Matthey in 50% H₂O

### Gemischter C16-20-Guerbetalkohol ex Octanol/Decanol

### Vergleichsbeispiel 1

Ein Reaktionsgemisch aus 2540g eines Gemisches aus Octan-1-ol und Decan-1-ol, 70 g KOH (50%ig) sowie 75g eines Gemisches aus Octanal und Decanal wurden unter Rühren und leichtem Überdruck auf 235°C erhitzt und 4 Stunden lang bei dieser Temperatur gerührt. Das bei der Reaktion entstandene Wasser wurde entfernt. Nach Abkühlen des Reaktionsgemisches wurde ein Reaktionsgemisch mit 64% Guerbetalkoholen erhalten.

### Vergleichsbeispiel 1a

Ein Reaktionsgemisch aus 2540g eines Gemisches aus Octan-1-ol und Decan-1-ol, 70 g KOH (50%ig), 75g eines Gemisches aus Octanal und Decanal und 5 g Pd/C wurden unter Rühren und leichtem Überdruck auf 225°C erhitzt und 2 Stunden lang bei dieser Temperatur gerührt. Das bei der Reaktion entstandene Wasser wurde entfernt. Nach Abkühlen des Reaktionsgemisches wurde ein Reaktionsgemisch mit 70 % Guerbetalkoholen erhalten.

### Vergleichsbeispiel 1b

Ein Reaktionsgemisch aus 2540g eines Gemisches aus Octan-1-ol und Decan-1-ol, 70 g KOH (50%ig), 75g eines Gemisches aus Octanal und Decanal und 5 g Pd/C wurden unter Rühren und leichtem Überdruck auf 200°C erhitzt und 4 Stunden lang bei dieser Temperatur gerührt. Das bei der Reaktion entstandene Wasser wurde entfernt. Nach Abkühlen des Reaktionsgemisches wurde ein Reaktionsgemisch mit 64 % Guerbetalkoholen erhalten.

### C10-Guerbet-Alkohol (Propylheptanol) ex Pentan-1-ol

### Vergleichsbeispiel 2

Ein Reaktionsgemisch aus 3100g Pentan-1-ol und 92g Pentanal sowie 56g KOH (50%ig) wurde auf 220°C aufgeheizt und bei einem sich einstellenden Eigendruck von 7 bar 22 Stunden unter Rühren gefahren. Nach Abkühlen fand sich ein Guerbetalkohol-Anteil von 30%.

### Vergleichsbeispiel 2

Ein Reaktionsgemisch aus 3100g Pentan-1-ol und 92g Pentanal, 56g KOH (50%ig) und 5 g Pd/C wurde auf 195°C aufgeheizt und bei einem sich einstellenden Eigendruck von 5,5 bar 5 Stunden unter Rühren gefahren. Nach Abkühlen fand sich ein Guerbetalkohol-Anteil von 30%.

### C10-Guerbet-Alkohol ex Isoamylalkohol

### Beispiel 3

Ein Reaktionsgemisch aus 2500g 3-Methyl-1-Butanol, 75g 3-Methyl-1-Butanal, 4 g Pd/C wurde auf 180°C aufgeheizt, 320g KOH (50%ig) portionsweise zudosiert und mit einer Druckrampe von 4,6 auf 1,4 bar 18 Stunden unter Rühren gefahren. Nach Abkühlen fand sich ein Guerbetalkohol-Anteil von 75%.

### C12-Guerbet-Alkohol ex Hexan-1-ol

### Vergleichsbeispiel 4

Ein Reaktionsgemisch aus 2500g Hexan-1-ol, 75g Hexanal, 4 g Pd/C wurde auf 210°C aufgeheizt, 80g KOH (50%ig) portionsweise zudosiert und mit einer Druckrampe von 5 auf 3 bar 6 Stunden unter Rühren gefahren. Nach Abkühlen fand sich ein Guerbetalkohol-Anteil von 38%.

## Patentansprüche

1. Verfahren zur Dimerisierung von Alkoholen im Sinne einer Guerbet-Reaktion, bei dem man 3-Methyl-butan-1-ol als Monoalkohol (A), in Gegenwart
(a) einer Base (B),
(b) einer Carbonylverbindung (C) und
(c) von Palladium als Hydrierkatalysators (H) wobei das Metall in elementarer Form vorliegen muss,
umsetzt.

2. Verfahren nach Anspruch 1, wobei man als Base (B) eine Verbindung einsetzt, die ausgewählt ist aus der Gruppe der Alkali- und Erdalkalihydroxide, Alkali- und Erdalkaliarbonate, Alkoxide, Amide und Hydride.

3. Verfahren nach Anspruch 1, wobei man als Base (B) eine Verbindung einsetzt, bei der Kalium als Kation fungiert.

4. Verfahren nach Anspruch 3, wobei man Kaliumhydroxid als Base (B) einsetzt.

5. Verfahren nach einem der Ansprüche 1 bis 4, wobei das Metall des Hydrierkatalysators (H) In eine hydrophobe Umgebung eingebettet ist.

6. Verfahren nach einem der Ansprüche 1 bis 4, wobei man Palladium auf Kohle (Pd/C) als Hydrierkatalysator (H) einsetzt.

7. Verfahren nach einem der Ansprüche 1 bis 6, wobei man die Umsetzung im Temperaturbereich von 140 °C bis 230 °C durchführt.

8. Verfahren nach einem der Ansprüche 1 bis 7, wobei man die Umsetzung bei Drücken im Bereich von 0,1 bis 6 bar durchführt.

## Claims

1. A process for dimerizing alcohols in the manner of a Guerbet reaction, in which 3-methylbutan-1-ol as the monoalcohol (A) is converted in the presence of
(a) a base (B),
(b) a carbonyl compound (C) and
(c) of palladium as hydrogenation catalyst (H), where the metal must be present in elemental form.

2. The process according to claim 1, wherein the base (B) used is a compound selected from the group of the alkali metal and alkaline earth metal hydroxides, alkali metal and alkaline earth metal carbonates, alkoxides, amides and hydrides.

3. The process according to claim 1, wherein the base (B) used is a compound in which potassium functions as the cation.

4. The process according to claim 3, wherein potassium hydroxide is used as the base (B).

5. The process according to any of claims 1 to 4, wherein the metal of the hydrogenation catalyst (H) is embedded in a hydrophobic environment.

6. The process according to any of claims 1 to 4, wherein palladium on carbon (Pd/C) is used as the hydrogenation catalyst (H).

7. The process according to any of claims 1 to 6, wherein the reaction is performed within the temperature range from 140°C to 230°C.

8. The process according to any of claims 1 to 7, wherein the reaction is performed at pressures in the range from 0.1 to 6 bar.

## Revendications

1. Procédé pour la dimérisation d'alcools dans le sens d'une réaction de Guerbet, dans lequel on transforme du 3-méthylbutan-1-ol en tant que monoalcool (A), en présence
(a) d'une base (B),
(b) d'un composé carbonyle (C) et
(c) de palladium en tant que catalyseur d'hydrogénation (H), le métal devant se trouver sous forme élémentaire.

2. Procédé selon la revendication 1, un composé, choisi dans le groupe formé par les hydroxydes de métal alcalin et de métal alcalino-terreux, les carbonates de métal alcalin et de métal alcalino-terreux, les alcoxydes, les amides et les hydrures, étant utilisé comme base.

3. Procédé selon la revendication 1, un composé dans lequel le potassium fonctionne comme cation étant utilisé comme base (B).

4. Procédé selon la revendication 3, de l'hydroxyde de potassium étant utilisé comme base (B).

5. Procédé selon l'une quelconque des revendications 1 à 4, le métal du catalyseur d'hydrogénation (H) étant enrobé dans un environnement hydrophobe.

6. Procédé selon l'une quelconque des revendications 1 à 4, du palladium sur carbone (Pd/C) étant utilisé comme catalyseur d'hydrogénation (H).

7. Procédé selon l'une quelconque des revendications 1 à 6, la transformation étant réalisée dans la plage de température de 140 à 230°C.

8. Procédé selon l'une quelconque des revendications 1 à 7, la transformation étant réalisée à des pressions dans la plage de 0,1 à 6 bars.
